# EUROPÄISCHE PATENTSCHRIFT

(11) **EP 2 801 383 B1**
(45) Veröffentlichungstag und Bekanntmachung des Hinweises auf die Patenterteilung: **03.02.2016**
(21) Anmeldenummer: 14167370.7
(22) Anmeldetag: 07.05.2014
(51) Int. Cl.: A61M 5/315

(54) **Drehspindelspritze**
Rotating spindle syringe
Seringue à tige rotative

(30) Priorität: 07.05.2013 EP 13166888; 10.01.2014 DE 102014100266
(43) Veröffentlichungstag der Anmeldung: 12.11.2014
(73) Patentinhaber: Ivoclar Vivadent AG, 9494 Schaan (LI)
(72) Erfinder: Müller, Frank, 6800 Feldkirch (AT); Vocke, Lutz, 9400 Rorschach (CH)
(74) Vertreter: Baronetzky, Klaus

(56) Entgegenhaltungen:
- DE-C1- 19 900 792

## Beschreibung

Die Erfindung betrifft eine Drehspindelspritze, gemäß dem Oberbegriff von Anspruch 1.

Eine derartige Drehspindelspritze ist beispielsweise aus der DE 199 00 792 C1 bekannt. Bei dieser Drehspindelspritze ist eine Betätigung zur Materialabgabe über ein Verdrehen eines Rändelrads möglich. Ferner lässt sich eine translatorische Bewegung durch Zurückziehen über den dortigen Deckel realisieren.

Diese Lösung ist jedoch ungeeignet, wenn es gilt, rasch eine erhebliche Materialmenge des Materials, das in der Drehspindelspritze aufgenommen ist, abzugeben.

Aus der DE 1 992 767 U1 ist eine Vorrichtung zum Feindosieren bekannt, die Flüssigkeit abgeben kann, und zwar über die Betätigung eines Kolbens. Die Feindosierung lässt sich durch Verstellen einer Gewindekappe realisieren, die auf einen separaten Kolben wirkt.

Diese Lösung ist recht aufwendig herzustellen und weist nicht die übliche Form einer Spritze auf.

Demgegenüber liegt der Erfindung die Aufgabe zu Grunde, eine kompakte und ergonomisch gut handhabbare Drehspindelspritze gemäß dem Oberbegriff von Anspruch 1 zu schaffen, die sowohl für die Feindosierung als auch für die rasche Materialabgabe geeignet ist.

Diese Aufgabe wird erfindungsgemäß durch Anspruch 1 gelöst. Vorteilhafte Weiterbildungen ergeben sich aus den Unteransprüchen.

Die erfindungsgemäße Lösung zeichnet sich zunächst dadurch aus, dass ein kompakter Spritzenkörper mit einem Gehäuse, einer Auslasskanüle und einem Stößel realisiert ist, der intutitiv bedienbar ist. Durch Druck auf den Stößel, der entweder unmittelbar als Kolben ausgebildet ist oder auf ein Kolben wirkt, lässt sich rasch und unkompliziert Material aus der Auslasskanüle ausdrücken. Zusätzlich oder alternativ lässt sich durch Verdrehen eines Drehelements, das erfindungsgemäß auf ein Mitnehmer und damit indirekt auf den Kolben wirkt, eine Feindosierung realisieren. Es ist sogar eine Einhandbedienung möglich, nämlich dergestalt, dass der Benutzer eine Außenhülse, gegenüber welcher das Drehelement verdrehbar ist, zwischen den drei hinteren Fingern und einer Hand hält und dann das Drehelement über Daumen und Zeigefinger gegenüber der Außenhülse verdreht.

Erfindungsgemäß ist es vorgesehen, dass der Mitnehmer auf den Kolben wirkt.

Der Mitnehmer bewegt sich bei Drehungen des Drehelements translatorisch, also in axialer Richtung, bezogen auf den Zylinder, der das Material aufnimmt und beispielsweise bezogen auf die Außenhülse. Bei Betätigung des Stößels oder Kolbens für die rasche Materialabgabe erfolgt eine translatorische Relativbewegung zwischen Mitnehmer und Kolben. Durch die Einräumung dieser Möglichkeit ist die doppelte Verwendbarkeit der erfindungsgemäßen Drehspindelspritze für die rasche.Materialabgabe einerseits und für die Feindosierung andererseits gewährleistet.

Bei Betätigung des Drehelements kann nach Wahl des Benutzers feindosiert Material abgegeben werden. Wahlweise kann aber auch bei Druck auf einer Handauflage des Kolbens oder eines Stößel des Kolbens Material abgegeben werden.

Erfindungsgemäß besonders günstig ist es, dass durch die Verlagerung des Mitnehmers über das Drehelement in den rückwärtigen Bereich der Drehspindelspritze - bei voller Spritze - der volle Bewegungsweg für die Betätigung des Mitnehmers für das Ausdrücken des Materials zur Verfügung steht. Hierzu weist das Drehelement ein entsprechend langes Innengewinde auf, das mit einem entsprechend geformten Außengewinde des Mitnehmers im Gewindeeingriff steht.

Der Mitnehmer ist drehfest gegenüber der Außenhülse und dem Zylinder angeordnet und verschiebt beim Drehen des Drehelements den Kolben nach vorne, um so Material auszudrücken. Das Verschieben kann entweder direkt erfolgen, dergestalt, dass der Mitnehmer in Reibschluss mit dem Kolben ist, oder indirekt, dergestalt, dass der Mitnehmer auf den Kolben wirkt und hierzu an ihm anliegt, um ihn translatorisch zu bewegen.

Im ersten Fall lässt sich der Kolben durch den Mitnehmer unter Überwindung des Reibschlusses hindurchschieben, um rasch Material auszudrücken. Im zweiten Fall ist der Kolben kompakt ausgebildet und von einem Stößel betätigbar, der den Mitnehmer durchtritt, ohne dass ein Reibschluss erforderlich wäre.

In einer modifizierten Ausgestaltung der erfindungsgemäßen Drehspindelspritze ist es vorgesehen, den rückwärtigen Teil des Spritzenkörpers als Drehelement auszubilden, oder aber gegebenenfalls, dass die gesamte Außenfläche des Spritzenkörpers. In letzterem Fall erfolgt das Verdrehen gegenüber dem Kolben, der dann drehfest an dem Zylinder geführt ist. In beiden Fällen ist die Realisierung recht einfach und ohne Aufwand möglich; im ersten Fall lassen sich Zylinder und Außenhülse auch einstückig ausgestalten, was automatisch eine drehfeste Verbindung ergibt.

Gemäß der Grundüberlegung der vorliegenden Erfindung sind Zylinder, Außenhülse und Mitnehmer in geeigneter Weise drehfest miteinander verbunden, wobei eine drehfeste Verbindung zwischen dem Kolben und dem Zylinder nicht unbedingt erforderlich ist. Gegenüber dem Spritzenkörper im Übrigen ist das Drehelement verdrehbar, aber in axialer Richtung fixiert gelagert, so dass stets ein Verdrehen des Drehelements zu einer axialen Bewegung des Mitnehmers führt.

Erfindungsgemäß besonders günstig ist es, dass durch die Riffelung des Drehelements auf der Außenseite, die auch als Aufrauung oder in beliebiger anderer geeigneter Weise ausgebildet sein kann, gepaart mit der Handauflage am Stößel und einer entsprechenden Fingerauflage am rückwärtigen Ende der Außenhülse die intuitive Bedienbarkeit der erfindungsgemäßen Drehspindelspritze sichergestellt ist.

Der Benutzer nimmt gleichsam automatisch die Drehspindelspritze mit zwei Fingern an der Fingerauflage und drückt dann mit dem Daumen oder Handballen auf die Handauflage, wenn er rasch Material ausdrücken will. Andererseits kann er auch entweder mit der anderen Hand oder aber auch der gleichen den Bereich des Drehelements fassen und verdrehen. Die Verdrehbarkeit kann gewünschtenfalls auch zusätzlich durch eine an der Außenfläche angebrachten Pfeil, der in Drehrichtung weist, noch deutlicher gemacht werden.

Erfindungsgemäß günstig ist es auch, dass das Gehäuse, also der Spritzenkörper in beliebiger geeigneter Weise eingefärbt werden kann. Beispielsweise lassen sich auch Stößel bzw. Kolben und der Spritzenkörper im Übrigen unterschiedlich färben, um das betreffende in der Drehspindelspritze je aufgenommene Material zu symbolisieren. Es ist auch möglich, über einen transparenten Spritzenkörper einen visuellen Einblick auf das zu exprimierende Material zu gewähren.

In weiterer vorteilhafter Ausgestaltung ist ein Sichtfenster vorgesehen, das den Füllzustand des Materials in der Drehspindelspritze deutlich werden lässt. Alternativ kann auch am Kolben oder an einer sonstigen geeigneten Stelle eine Skala oder sonstige Markierungen realisiert sein, die den Füllzustand der Drehspindelspritze erkennen lässt.

Weitere Vorteile, Einzelheiten und Merkmale ergeben sich aus der nachfolgenden Beschreibung zweier Ausführungsbeispiele anhand der Zeichnungen.

Es zeigen:
- Fig. 1: einen schematischen Schnitt durch eine Ausführungsform einer erfindungsgemäßen Drehspindelspritze; und
- Fig. 2: einen schematischen Schnitt durch eine weitere Ausführungsform einer erfindungsgemäßen Drehspindelspritze

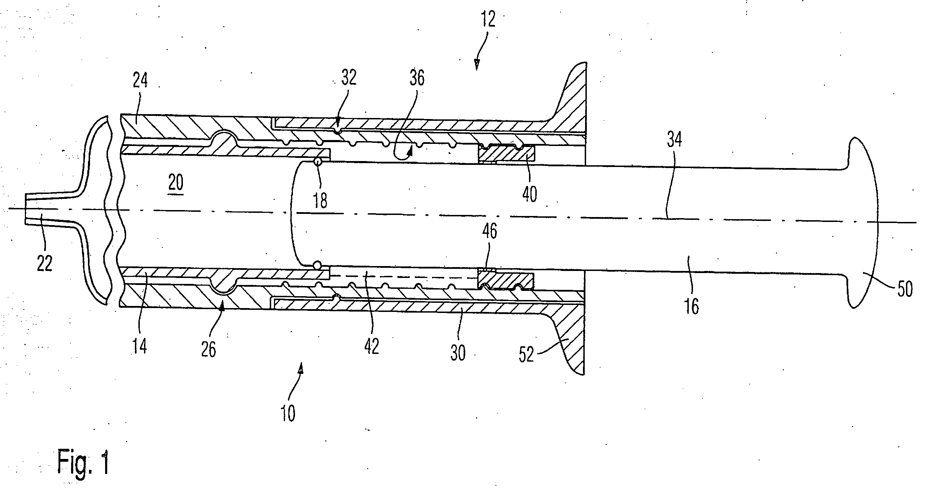

Die in Fig. 1 dargestellte Drehspindelspritze 10 weist einen Spritzenkörper 12 auf, der mehrteilig ausgebildet ist. Ein Zylinder 14 nimmt einen Kolben 16 in an sich bekannter Weise translatorisch beweglich auf. Über einen ebenfalls an sich bekannten Dichtring 18 ist eine Abdichtung beim Einschieben des Kolbens 16 in den Zylinder 14 gewährleistet. In dem Zylinder 14 ist Material 20 aufgenommen, das über eine Auslasskanüle 22 ausgedrückt werden kann.

Der Zylinder 14 lagert ein Drehelement 24, das axial fix, jedoch drehbeweglich an dem Zylinder 14 gelagert ist. Hierzu weisen der Zylinder 14 und das Drehelement 24 eine Nut- / Federverbindung 26 auf. Im Beispielsfall ist die Nut in dem Drehelement 24 und die Feder an den Zylinder 14 vorgesehen, wobei es sich versteht, dass die Ausgestaltung auch vertauscht sein kann.

An dem Drehelement 24 ist eine Außenhülse 30 ebenfalls drehbeweglich aber axial fix gelagert. Hierzu ist eine entsprechende Nut- / Federverbindung 32 zwischen dem Drehelement 24 und der Außenhülse 30 ausgebildet, so dass indirekt die axial fixe Lagerung der Außenhülse 30 an dem Zylinder 14 erreicht ist.

Eine Achse 34 verläuft durch den Kolben 16 und bis durch die Auslasskanüle 22 hindurch, und die erfindungsgemäße Drehspindelspritze ist punktsymetrisch zu dieser Achse 34 ausgebildet.

Erfindungsgemäß ist das Drehelement 24 mit einem Innengewinde 36 versehen. Das Innengewinde 36 steht im Gewindeeingriff mit einem Mitnehmer 40. Er ist axial verschieblich an dem Kolben 16 gelagert. Über eine Winkelanlage, die im dargestellten Ausführungsbeispiel als schwertförmiger Zylinderfortsatz 42 ausgebildet ist, ist er drehfest gegen den Zylinder 14 abgestützt. Über diese Drehmomentabstützung wird ein Verdrehen des Mitnehmers 40 gegenüber dem Zylinder 14 verhindert.

Beim Verdrehen des Drehelements 24 gegenüber dem Spritzenkörper 12 im Übrigen wird hierdurch der Mitnehmer 40 nicht verdreht, sondern aufgrund des Gewindeeingriffs in axialer Richtung bewegt.

Der Mitnehmer 40 ist über einen Anlagebereich 46 an dem Kolben 16 gelagert. Er befindet sich in Reibschluss mit dem Kolben. Bei Betätigung des Drehelements 24 wird daher der Kolben 16 unter Beibehaltung des Reibschlusses zusammen mit dem Mitnehmer 40 in Richtung Auslasskanüle 22 bewegt, so dass Material 20 die Auslasskanüle verlässt.

Wenn rasch Material ausgedrückt werden soll, wird eine Handauflage 50 des Kolbens betätigt, und zwar gegen eine Fingerauflage 52 der Außenhülse 30. Hierdurch rutscht der Kolben 16 im Anlagebereich 46 an dem Mitnehmer 40 entlang bzw. durch diesen hindurch, und Material 20 wird ebenfalls ausgedrückt, und zwar recht rasch.

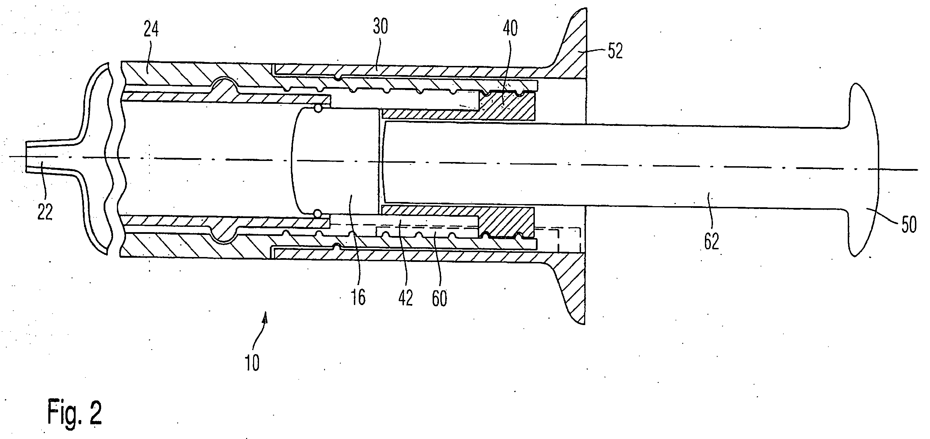

Um eine Drehabstützung zwischen dem Drehelement 24 und dem Spritzenkörper 12 im Übrigen auch an der Außenhülse 30 zu gewährleisten, ist diese bevorzugt ebenfalls drehfest mit dem Zylinder 14 und damit dem Mitnehmer 40 verbunden. Eine hierfür möglich Realisierung ist über den Drehanschlag 60 aus Fig. 2 ersichtlich. Der Drehanschlag 60 stützt sich gegen den schwertförmige Zylinderfortsatz 42 ab und bildet insofern eine drehfeste Verbindung, auch wenn sich der Mitnehmer 40 an dem schwertförmigen Zylinderfortsatz 42 etwas Spiel hat.

Fig. 2 weist im Übrigen eine modifierte Ausgestaltung des Mitnehmers 40 und des Kolbens 16 auf. Der Mitnehmer 40 wirkt in axialer Richtung auf den nach der Art eines Flachzylinders ausgebildeten Kolben 16, an dessen Außenumfang. Er übt Druck in Ausdruckrichtung, also in Richtung der Kanüle 22 aus, wenn das Drehelement 24 gedreht wird und damit der Mitnehmer 40 axial verschoben wird.

Auch bei dieser Ausführungsform ist der Mitnehmer 40 mit einer Durchtrittsausnehmung versehen, diese durchdringt ein Stößel 62, der an den Kolben 16 anliegt und diesen vorschieben kann, wenn eine rasche Materialabgabe erwünscht ist.

Die aus Fig. 2 ersichtliche Ausgestaltung weist auch den besonderen Vorzug auf, dass die rasche und feinfühlige Materialabgabe unabhängig voneinander erfolgen kann. Eine Präzisionsfertigung zur Bereitstellung einer exakten Reibkraft ist nicht erforderlich.

Demgegenüber weist die Ausführungsform gemäß Fig. 1 den besonderen Vorteil auf, dass das Drehelement nicht nachgedreht werden muss, wenn über den Kolben 16 rasch Material abgegeben worden ist.

## Patentansprüche

1. Drehspindelspritze, mit einem Drehelement (24) mit Gewinde, das mit einem entsprechend geformten Außengewinde eines Mitnehmers (40) im Gewindeeingriff (40) steht, **dadurch gekennzeichnet, dass** der Mitnehmer (40) auf einen Kolben (16) wirkt und gegenüber diesen translatorisch beweglich gelagert ist und dass die Drehspindelspritze (10) sowohl bei Betätigung des Drehelements (24) als auch bei Druck auf eine Handauflage (50) des Kolbens (16) oder eines Stößels (62) des Kolbens (16) Material abgibt.

2. Drehspindelspritze nach Anspruch 1, **dadurch gekennzeichnet, dass** das Drehelement (24) einer Auslasskanüle (22) der Drehspindelspritze (10) benachbart angeordnet ist und gegenüber einem Spritzenkörper (12) im Übrigen, insbesondere einer Außenhülse (30), drehbar ist.

3. Drehspindelspritze nach einem der vorhergehenden Ansprüche, **dadurch gekennzeichnet, dass** das Drehelement (24) mit seiner Außenfläche sich in Verlängerung des Spritzengehäuses im Übrigen erstreckt, insbesondere bündig zu diesem und mit einer profilierten oder aufgerauten Oberfläche.

4. Drehspindelspritze nach einem der vorhergehenden Ansprüche, **dadurch gekennzeichnet, dass** der Kolben (16) eine Auflage (50) aufweist, bei Druck auf welche in der Drehspindelspritze (10) enthaltenes Material ausdrückbar ist.

5. Drehspindelspritze nach einem der vorhergehenden Ansprüche, **dadurch gekennzeichnet, dass** das Drehelement (24) über den Mitnehmer (40) den Kolben (16) zum Ausdrücken des Materials aus der Drehspindelspritze (10) bewegt.

6. Drehspindelspritze nach einem der vorhergehenden Ansprüche, **dadurch gekennzeichnet, dass** der Mitnehmer (40) durch Anlage, insbesondere reibschlüssig, auf einen Kolben (16) wirkt, welcher Kolben (16) wahlweise durch Betätigung eines Stößels (62) oder des Mitnehmers (40) in Richtung Auslassende der Drehspindelspritze (10) bewegbar ist.

7. Drehspindelspritze nach einem der vorhergehenden Ansprüche, **dadurch gekennzeichnet, dass** der Mitnehmer (40) über eine Verdrehsicherung, insbesondere einen schwertförmigen Zylinderfortsatz (42), drehfest gehalten ist, insbesondere an einem Innenzylinder in welchem der Kolben (16) geführt ist, und in Gewindeeingriff mit dem Drehelement (24) steht.

8. Drehspindelspritze nach einem der vorhergehenden Ansprüche, **dadurch gekennzeichnet, dass** der Mitnehmer (40) in Reibschluss mit dem Kolben (16) verbunden ist und diesen bei der Drehung des Drehelements (24) in Auslassrichtung mitnimmt.

9. Drehspindelspritze nach einem der vorhergehenden Ansprüche, **dadurch gekennzeichnet, dass** der Mitnehmer (40) in Reibschluss mit dem Kolben (16) angeordnet ist und drehfest an einen schwertförmigen Zylinderfortsatz (42) geführt ist.

10. Drehspindelspritze nach einem der vorhergehenden Ansprüche, **dadurch gekennzeichnet, dass** der Kolben (16) gegenüber dem Drehmitnehmer, insbesondere unter Überwindung eines Reibschlusses, verschieblich gelagert ist.

11. Drehspindelspritze nach einem der vorhergehenden Ansprüche, **dadurch gekennzeichnet, dass** der Zylinder (14) an dem Drehmitnahmeelement verdrehbar, jedoch axial fest, gelagert ist, und insbesondere an dieser Stelle eingeschnappt ist.

12. Drehspindelspritze nach einem der vorhergehenden Ansprüche, **dadurch gekennzeichnet, dass** eine Füllstandsanzeige an dem Kolben (16) und/oder der Außenhülse angebracht ist, insbesondere durch eine Markierung oder eine Skala an dem Kolben (16), die über ein Fenster in der Außenhülse sichtbar ist.

13. Drehspindelspritze nach einem der vorhergehenden Ansprüche, **dadurch gekennzeichnet, dass** der Drehmitnehmer bei mit Material geladener Drehspindelspritze (10) im rückwärtigen Teil der Drehspindelspritze (10) ist und von dem Drehelement (24) nach vorne, also zur Auslasskanüle (22) hin, gezogen wird, wenn das Drehelement (24) betätigt wird.

14. Drehspindelspritze nach einem der vorhergehenden Ansprüche, dadurch gekennezeichnet, dass bei Betätigung des Drehelements (24) der Mitnehmer (40) auf einen Kolben (16) zur Materialabgabe durch die Auslasskanüle (22) drückt, und dass ein Stößel (62) in oder an dem Mitnehmer (40) geführt ist und ebenfalls auf den Kolben (16) drückt, wenn durch Betätigung des Stößels (62) Material aus der Auslasskanüle (22) abgegeben wird.

## Claims

1. A rotary-spindle syringe, comprising a rotary element (24) with a thread which is in threaded engagement with a correspondingly shaped external thread of a driver (40), **characterized in that** the driver (40) acts on a piston (16) and is mounted in a translationally moveable manner relative to the piston, and **in that** the rotary-spindle syringe (10) dispenses material both upon actuation of the rotary element (24) as well as upon application of pressure to a hand support (50) of the piston (16) or a plunger (62) of the piston (16).

2. The rotary-spindle syringe according to claim 1, **characterized in that** the rotary element (24) is disposed adjacent to an outlet cannula (22) of the rotary-spindle syringe (10) and is rotatable relative to a remaining syringe body (12), in particular to an outer sleeve (30).

3. The rotary-spindle syringe according to one of the preceding claims, **characterized in that** the outer surface of the rotary element (24) which is a profiled or roughened surface extends in extension to the remaining syringe housing, in particular flush with it.

4. The rotary-spindle syringe according to one of the preceding claims, **characterized in that** the piston (16) comprises a support (50) and upon pressure to it material contained in the rotary-spindle syringe (10) can be expressed.

5. The rotary-spindle syringe according to one of the preceding claims, **characterized in that** the rotary element (24) moves the piston (16) via the driver (40) for expressing the material from the rotary-spindle syringe (10).

6. The rotary-spindle syringe according to one of the preceding claims, **characterized in that** the driver (40) acts on a piston (16) through contact, in particular by friction, which piston (16) can be moved towards the outlet end of the rotary-spindle syringe (10) either upon actuation of a plunger (62) or of the driver (40).

7. The rotary-spindle syringe according to one of the preceding claims, **characterized in that** the driver (40) is held in a torque-proof manner, in particular at an inner cylinder along which the piston (16) is guided, via an anti-rotation stop, in particular a sword-shaped cylinder extension (42), and is in threaded engagement with the rotary element (24).

8. The rotary-spindle syringe according to one of the preceding claims, **characterized in that** the driver (40) is connected with the piston (16) through frictional contact and carries it along upon turning the rotary element (24) in the outlet direction.

9. The rotary-spindle syringe according to one of the preceding claims, **characterized in that** the driver (40) is disposed in frictional contact with the piston (16) and is guided along a sword-shaped cylinder extension (42) in a torque-proof manner.

10. The rotary-spindle syringe according to one of the preceding claims, **characterized in that** the piston (16) is mounted slidably relative to the rotary driver, in particular by overcoming frictional contact.

11. The rotary-spindle syringe according to one of the preceding claims, **characterized in that** the cylinder (14) is mounted pivotably to the rotary driver element, yet in an axially fixed fashion, and is in particular snap fitted into that position.

12. The rotary-spindle syringe according to one of the preceding claims, **characterized in that** a level indicator is attached to the piston (16) and/or the outer sleeve, in particular by means of a marking or a scale at the piston (16) which is visible through a window in the outer sleeve.

13. The rotary-spindle syringe according to one of the preceding claims, **characterized in that** the rotary driver is in the rear part of the rotary-spindle syringe (10) when the rotary-spindle syringe (10) is filled with material, and is drawn to the front, that is to say towards the outlet cannula (22), by the rotary element (24) upon actuation of the rotary element (24).

14. The rotary-spindle syringe according to one of the preceding claims, **characterized in that** upon actuation of the rotary element (24) the driver (40) applies pressure to a piston (16) for dispensing material through the outlet cannula (22), and **in that** a plunger (62) is guided in or at the driver (40) and also applies pressure to the piston (16) when material is dispensed from the outlet cannula (22) by actuating the plunger (62).

## Revendications

1. Seringue à broche rotative avec un élément rotatif (24) avec filetage, qui est en prise vissée avec un filetage extérieur d'un entraîneur (40) formé de façon correspondante, **caractérisé en ce que** l'entraîneur (40) agit sur un vérin (16) et est monté mobile en translation en face de celui-ci et que la seringue à broche rotative (10) fournit du matériel lors de l'actionnement de l'élément rotatif (24) ainsi que lors de la pression sur un support de la main (50) du vérin (16) ou d'un poussoir (62) du vérin (16).

2. Seringue à broche rotative selon la revendication 1, **caractérisée en ce que** l'élément rotatif (24) est disposé avoisinant une canule d'évacuation (22) de la seringue à broche rotative (10) et en outre peut être tourné par rapport à un corps de seringue(12), en particulier un manchon extérieur (30).

3. Seringue à broche rotative selon l'une des revendications précédentes, **caractérisée en ce que** l'élément rotatif (24) s'étend en outre avec sa surface extérieure en extension du corps de la seringue, en particulier à fleur avec celui-ci et avec une surface rugueuse ou profilée.

4. Seringue à broche rotative selon l'une des revendications précédentes, **caractérisée en ce que** le vérin (16) est doté d'un support (50), et lors de la pression sur celui-ci le matériel contenu dans la seringue à broche rotative (10) est exprimable.

5. Seringue à broche rotative après l'une des revendications précédentes, **caractérisée en ce que** l'élément rotatif (24) déplace le vérin (16) par le biais de l'entraîneur (40) pour exprimer le matériau de la seringue à broche rotative (10).

6. Seringue à broche rotative selon l'une des revendications précédentes, **caractérisée en ce que** l'entraîneur (40) agit sur un vérin (16) par appui, en particulier par la friction, lequel vérin (16) peut être déplacé en actionnant soit un vérin soit l'entraîneur (40) en direction de l'extrémité de sortie de la seringue à broche rotative (10).

7. Seringue à broche rotative selon l'une des revendications précédentes, **caractérisée en ce que** l'entraîneur (40) par une sécurité anti-rotation, en particulier par une extension de cylindre en forme d'épée (42), est maintenu immobilisé en rotation, en particulier à un cylindre intérieur dans lequel le vérin (16) est conduit et se trouve en prise vissée avec l'élément rotatif (24).

8. Seringue à broche rotative selon l'une des revendications précédentes, **caractérisée en ce que** l'entraîneur (40) est maintenu en friction avec le vérin (16) et lors de la rotation de l'élément rotatif (24) entraîne celui-ci dans le sens de sortie.

9. Seringue à broche rotative selon l'une des revendications précédentes, **caractérisée en ce que** l'entraîneur (40) est disposé en friction avec le vérin (16) et, maintenu immobilisé en rotation, longe une extension de cylindre en forme d'épée (42).

10. Seringue à broche rotative selon l'une des revendications précédentes **caractérisée en ce que** le vérin (16) est monté de manière déplaçable par rapport à l'entraîneur rotatif, en particulier en surmontant une friction.

11. Seringue à broche rotative selon l'une des revendications précédentes, **caractérisée en ce que** le cylindre (14) est monté sur l'entraîneur rotatif de manière pivotable, cependant fixement axialement, et en particulier est encliqueté à cet endroit.

12. Seringue à broche rotative selon l'une des revendications précédentes, **caractérisé en ce que** d'un indicateur de niveau est apposé sur le vérin (16) et/ou le manchon extérieur par un marquage ou une échelle sur le vérin (16), qui est visible à travers une fenêtre sur le manchon.

13. Seringue à broche rotative selon l'une des revendications précédentes, **caractérisée en ce que** lorsque la seringue à broche rotative (10) est chargé avec du matériel, l'entraîneur rotatif se trouve dans la partie arrière de la seringue à broche rotative (10) et est poussé vers l'avant, c'est à dire vers la canule d'évacuation (22), par l'élément rotatif (24), lorsque l'élément rotatif (24) est actionné.

14. Seringue à broche rotative selon l'une des revendications précédentes, **caractérisée en ce que**, lors de l'actionnement de l'élément rotatif (24) l'entraîneur (40) appuie sur un vérin (16) pour le dégagement de matériel par la canule d'évacuation (22), et qu'un poussoir (62) est conduit dans ou le long de l'entraîneur (40) et appuie également sur le vérin (16), lorsque du matériel est dégagée de la canule d'évacuation (22) en appuyant sur le poussoir (62).
